# EUROPEAN PATENT SPECIFICATION

(11) **EP 0 686 018 B1**
(45) Date of publication and mention of the grant of the patent: **11.08.1999**
(21) Application number: 93921356.7
(22) Date of filing: 03.09.1993
(51) Int. Cl.: A61F 2/14

(54) **ASTIGMATIC CORRECTING INTRASTROMAL CORNEAL RING**
INTRASTROMALER, KORNEALER RING ZUR ASTIGMATISMUSKORREKTUR
ANNEAU CORNEEN INTRASTROMAL CORRIGEANT L'ASTIGMATISME

(30) Priority: 03.09.1992 US 939492
(43) Date of publication of application: 13.12.1995
(73) Proprietor: KERAVISION, INC., Fremont, California 94538-7353 (US)
(72) Inventor: SILVESTRINI, Thomas, A., Alamo, CA 95407 (US)
(74) Representative: Price, Nigel John King
(86) International application number: US9308345
(87) International publication number: WO9405232

(56) References cited:
- WO-A-88/10096
- WO-A-93/13724
- GB-A- 2 250 442
- US-A- 4 976 719
- US-A- 4 994 081
- US-A- 5 098 443
- US-A- 5 188 125
- DATABASE WPI Section PQ, Week 9345, Derwent Publications Ltd., London, GB; Class P, AN 93-358291 & SU-A-1 771 730 (ALMA-ATA DOCTORS TRAINING INST.)

## Description

### Field of the Invention

This invention is an intrastromal corneal ring ("ICR") which is not dimensionally uniform about the ring. It has at least one area, or, more typically, two or more raised areas (or areas of additional bulk, spaced apart from each other on the ring. This ring design, when introduced into the stroma and properly adjusted for position there, permits at least partial correction of astigmatism in the eye.

### Background of the Invention

Anomalies in the overall shape of the eye can cause visual disorders. Hyperopia ("farsightedness") occurs when the front-to-back distance in the eyeball is too small. In such a case, parallel rays originating greater than 6 metres (20 feet) from the eye focus behind the retina. In contrast, when the front-to-back distance of eyeball is too large, myopia ("nearsightedness") occurs and the focus of parallel rays entering the eye occurs in front of the retina. Astigmatism is a condition which occurs when the parallel rays of light do not focus to a single point within the eye, but rather focus to a region due to the fact that the cornea is aspherical and refracts light in a different meridian at different distances. Some degree of astigmatism in an eye is normal, but where the astigmatism is too pronounced, it must often be corrected.

Hyperopia, myopia, and astigmatism are usually corrected by glasses or contact lenses. Surgical methods for the correction of such disorders are known. These methods include radial keratotomy (see, e.g., U.S. Patents Nos. 4,815,463 and 4,688,570) and laser corneal ablation (see, e.g., U.S. Patent No. 4,941, 093).

Another method for correcting those disorders is through the implantation of polymeric rings in the eye's corneal stroma to change the curvature of the cornea. Previous work involving the implantation of polymethylmethacrylate (PMMA) rings, allograft corneal tissue and hydrogels is well documented. One of the ring devices involves a split ring design which is inserted into a channel previously dissected in the stromal layer of the cornea. The device uses a minimally invasive incision through which the channel for the implant is created and, finally, through which the implant is inserted.

U.S. Patent No. 4,452,235, to Reynolds, describes a method and apparatus for corneal curvature adjustment. The method involves inserting one end of a split end adjusting ring into the cornea of the eye and moving the ring in a circular path until its ends meet. The ends are thereafter adjusted relative to each other until the shape of the eye has assumed a desired curvature whereupon the ends are fixedly attached to maintain the desired curvature of the cornea.

WO-A-93/13724 published on 22/07/93 and relevant under Article 54/3/EPC, suggests the use of ICRs for the correction of astigmatism. That disclosure does not suggest the use of ICRs having the inventive shape to alleviate astigmatism in the eye.

WO-A-88/10096 discloses a ring in accordance with the preamble of claim 1.

According to the present invention there is provided a split polymeric ring having a cross-section, which ring is suitable for introduction into the stroma of a patient's eye and is adapted to substantially encircle the cornea, said ring having a first end, a second end, and an arcuate body extending therebetween, characterised in that the cross-section of said arcuate body has at least one area of comparatively larger dimension.

The following description and drawings disclose embodiments of astigmatic correcting intracorneal rings ("ACICR") which are in accordance with the present invention. These devices are inserted into the interlamellar region of the eye's stroma. For the purpose of alleviating astigmatism, the device is an improvement over previously existing ICRs. Unlike other ICRs which have a constant cross section when viewed through various cross-sections of the ring, the inventive ACICR typically has at least one region in which the cross section is thicker or the bulk of that region is more pronounced. Often the rings will have two or more regions at which the bulk is increased. By proper alignment of the larger regions of the ACICR with the eye's anomalies, the astigmatism may be alleviated.

The devices for forming the intrastromal pathway into which these ICRs may be placed is well known.

### Brief Description of the Drawings

Figure 1 is a schematic representation of a horizontal section of the eye.
Figure 2 is a schematic representation of the anterior portion of the eye showing the various layers of the cornea.
Figure 3 depicts the inventive ACICR as it resides within the cornea.
Figure 4 is a side cross-section of the ACICR within the cornea.
Figure 5 is a frontal view showing the placement of the ACICR within the eye.
Figure 6 is a frontal view of the ACICR having symmetrical areas of added bulk or cross-section.
Figure 7 is a frontal view of an ACICR having non-symmetric areas of added bulk or cross-section.
Figure 8 depicts a feature of the invention which allows the ACICR to be manipulated into proper position for astigmatism correction.
Figures 9A and 9B show a variation of the inventive ACICR using a composite ring in which a portion of the ring uses a swellable polymer.
Figure 10 shows an ACICR in which the thickness of the ring is varied.
Figure 11 shows an ACICR in which the ring is a composite of a stiff polymer and a swellable polymer.

### Description of the Invention

Prior to explaining the details of the inventive devices, a short explanation of the physiology of the eye is needed to appreciate the functional relationship of the device to the eye.

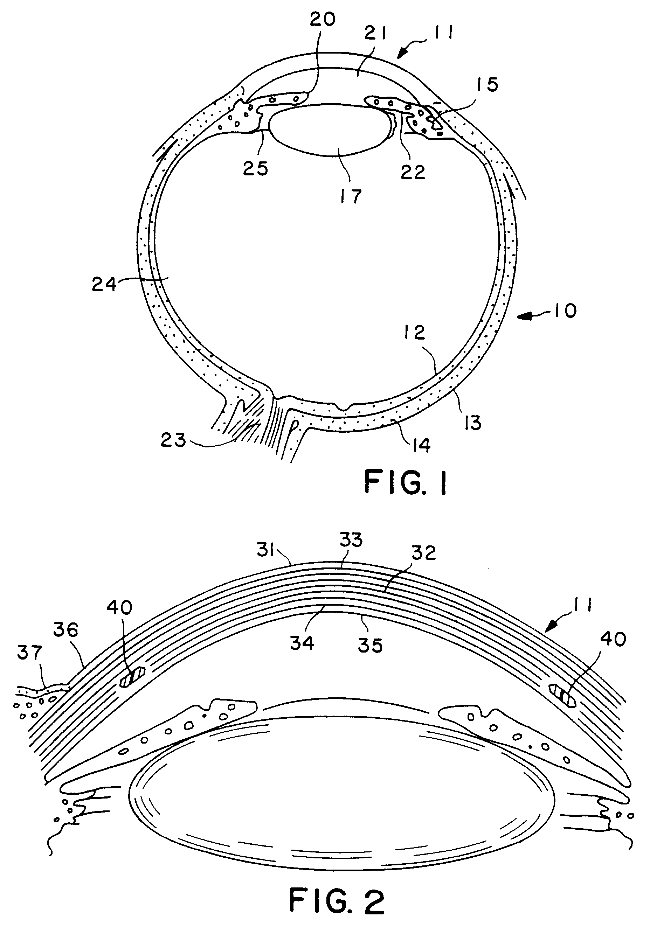

Figure 1 shows a horizontal section of the eye with the globe (10) of the eye resembling a sphere with an anterior bulged spherical portion representing the cornea (11).

The globe (10) of the eye consists of three concentric coverings enclosing the various transparent media through which the light must pass before reaching the light-sensitive retina (12). The outermost covering is a fibrous protective portion the posterior five-sixths of which is white and opaque and called the sclera (13), and sometimes referred to as the white of the eye where visible to the front. The anterior one-sixth of this outer layer is the transparent cornea (11).

A middle covering is mainly vascular and nutritive in function and is comprised of the choroid, ciliary body (15) and iris (20). The choroid (14) generally functions to maintain the retina (12). The ciliary body (15) is involved in suspending the lens (17) and accommodation of the lens. The iris (16) is the most anterior portion of the middle covering of the eye and is arranged in a frontal plane. It is a thin circular disc similar in function to the diaphragm of a camera, and is perforated near its center by a circular aperture called the pupil (20). The size of the pupil varies to regulate the amount of light which reaches the retina (12). It contracts also to accommodation, which serves to sharpen the focus by diminishing spherical aberration. The iris divides the space between the cornea (11) and the lens (17) into an anterior chamber (21) and posterior chamber. The innermost portion of covering is the retina (12), which is made up of nerve elements which form the true receptive portion for visual impressions. The retina (12) is a part of the brain arising as an outgrowth from the fore-brain, with the optic nerve (23) serving as a fiber tract connecting the retina part of the brain with the fore-brain. A layer of rods and cones, lying just beneath a pigmented epithelium on the anterior wall of the retina serve as visual cells or photoreceptors which transform physical energy (light) into nerve impulses. The vitreous body (24) is a transparent gelatinous mass which fills the posterior four-fifths of the globe (10). At its sides it supports the ciliary body (15) and the retina (12). A frontal saucer-shaped depression houses the lens. The lens (17) of the eye is a transparent biconvex body of crystalline appearance placed between the iris (16) and vitreous body (24). Its axial diameter varies markedly with accommodation. A ciliary zonule (25), consisting of transparent fibers passing between the ciliary body (15) and lens (17) serves to hold the lens (17) in position and enables the ciliary muscle to act on it.

Referring again to the cornea (11), this outermost fibrous transparent coating resembles a watch glass. Its curvature is somewhat greater than the rest of the globe and is ideally spherical in nature. However, often it is more curved in one meridian than another giving rise to astigmatism. A central third of the cornea is called the optical zone with a slight flattening taking place outwardly thereof as the cornea thickens towards its periphery. Most of the refraction of the eye takes place at the cornea.

Referring to Figure 2, a more detailed drawing of the anterior portion of the globe shows the various layers of the cornea (11) made up of an epithelium (31). Epithelial cells on the surface thereof function to maintain transparency of the cornea (11). These epithelial cells are rich in glycogen, enzymes, and acetylcholine and their activity regulates the corneal corpuscles and controls the transport of water and electrolytes through the lamellae of the stroma (32) of the cornea (11).

An anterior limiting lamina (33), referred to as Bowman's membrane or layer, is positioned between the epithelium (31) and the stroma (32) of the cornea. The stroma (32) is comprised of lamella having bands of fibrils parallel to each other and crossing the whole of the cornea. While most of the fibrous bands are parallel to the surface, some are oblique, especially anteriorly. A posterior limiting lamina (34) is referred to as Descemet's membrane. It is a strong membrane sharply defined from the stroma (32) and resistant to pathological processes of the cornea.

The endothelium (35) is the most posterior layer of the cornea and consists of a single layer of cells. The limbus (37) is the transition zone between the conjunctiva and sclera on the one hand and the cornea (11) on the other.

As has been explained above, most of the eye's refraction takes place at the outer portion of the cornea (12). The overall concept behind this invention is that by addition of selected amounts of bulk at the steeper portions of the anterior cornea or by inclusion of a non-uniform ring in radial tension, the anterior corneal surface will be forced into a generally spherical surface thereby correcting the undesired astigmatism.

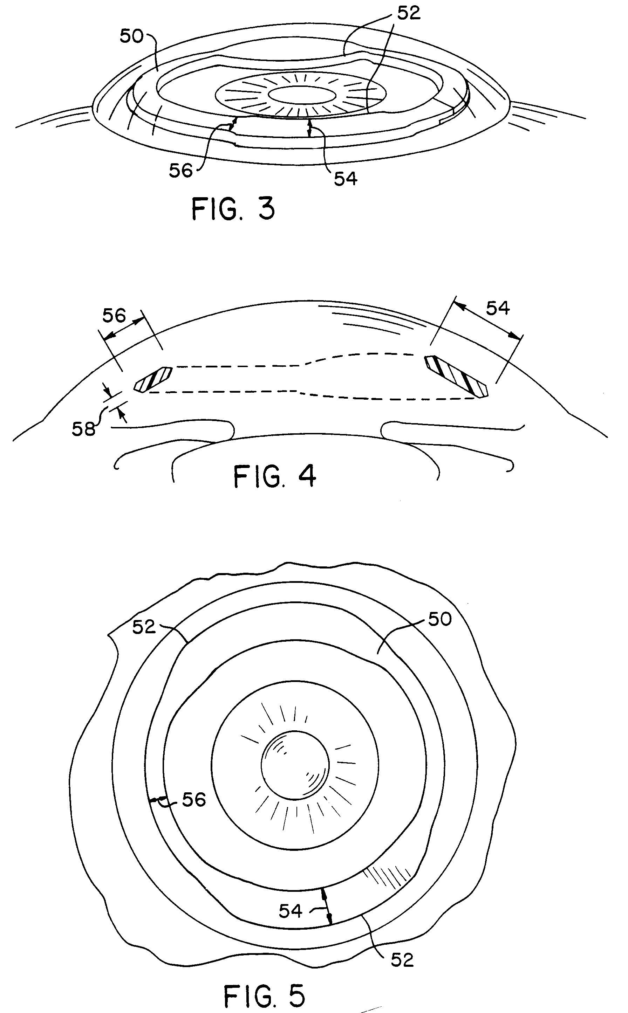

Figure 3 shows the placement of the non-regular ACICR within the cornea as discussed above. In this instance, the ring (50) has two regions (52) of added bulk or dimension. The larger regions in this instance have a larger relational width (54) than the local width of the narrower region (56) . See also the side view of the comparative widths in Figure 3. The relationship between the local widths (54) and (56), the thickness (58) at various positions of the ACICR, and their respective corrections, are known and may be determined from WO-A-93/13724 referred to above.

Figure 5 is a frontal view of the eye having an ACICR installed. The ring (50) has two regions of added dimension (52). Those regions of added dimension would be placed in the regions of the cornea having the steepest slope in an effort to correct the cornea to an approximate spherical, or at least regular, shape about its anterior surface.

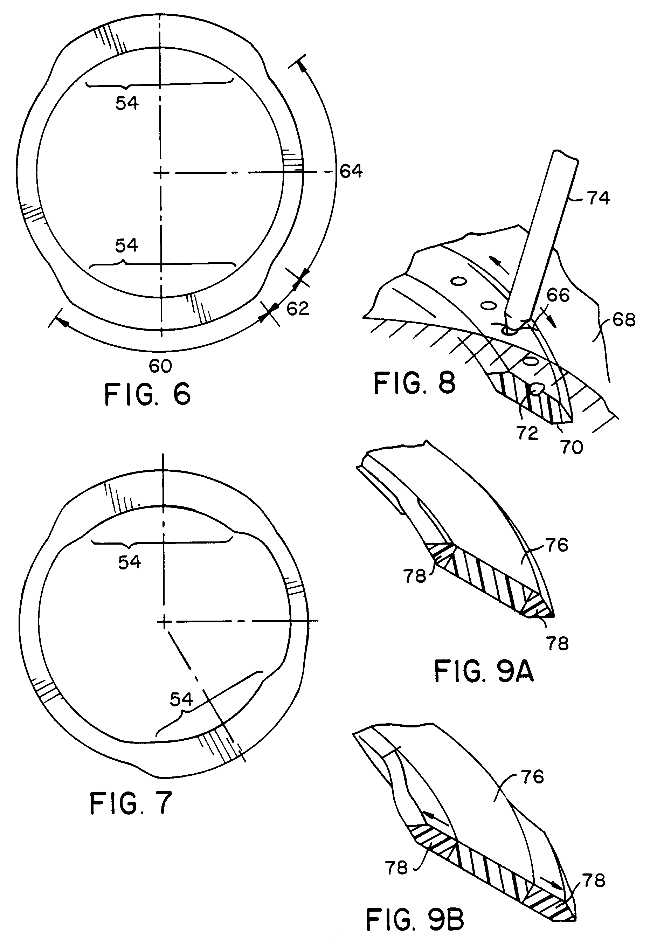

Figure 6 shows a frontal view of an ACICR in which the regions of added dimension are generally symmetrical. The regions of added dimension (54) are shown each to extend over a region of about 90° of the ACICR. Figure 6 also shows a transition zone (62) between the area of added bulk 60 and the comparatively thinner region at (64). The transition zone allows the ACICR to be inserted into an intrastromal channel with greater ease. We believe this permits installation of the ACICR with less trauma. The arc (64) of lesser dimension is shown in Figure 6. As with the local diameters (54) and (56) of the ACICR as shown in Figures 3 and 4, the percentage of arcs (60) and (64) and their respective relationship to each other are a function of the level of astigmatism to be corrected.

Figure 7 shows an ACICR with regions of enhanced dimension which are not placed symmetrically about the ring. Again, such a ring would be employed in an eye which did not have symmetrical astigmatism about a single axis. Such an ACICR would be employed with the intent to bring the corneal shape back into general spherical form.

The ring would be installed in the inner lamellar regions of the corneal stroma by any of the methods we have shown in the past to be suitable for such installation. Particularly desired is the process and its allied apparatus shown in WO-A-93/20763, published on 28/10/93 and relevant under Article 54/3/EPC. In general, the ring is installed in the following manner: a small radial incision is made at the radius in which the ring is ultimately to be installed about the cornea. A dissector in the form of a split ring and having a point suitable for producing an interlamellar channel or tunnel in the corneal stroma is introduced through the small incision and rotated in such a fashion that a generally circular channel is formed completely about the cornea. The dissector is then rotated in the opposite direction to withdraw it from the tunnel thus formed. An ACICR is then introduced into the circular channel and joined at its ends. ICRs of constant cross-section typically need no further adjustment other than, perhaps, to move the point of junction away from the region of corneal incision so to help assure that the junction is held together by the interlamellar tension of the cornea. However, with an ACICR, the relationship of the ring and the astigmatic aberration must be aligned so to allow the ACICR to perform its desired correction. In Figure 8, one such method for adjusting the position of the ACICR in the eye is shown. The radial incision (66) in cornea (68) which is used to introduce the ICR into the eye is shown. The ACICR (70) has a number of depressions (72) spaced about its upper surface. The position of the ACICR (70) is changed by engaging a generally pointed tool (74) into the depressions and slipping the ring (70) around in one direction or another until the regions of added dimension are in appropriate position for correcting the astigmatic aberrations of the cornea. Other variations on this will be apparent to those studying the need to adjust the position of these rings.

The materials used in these rings are typically stiff physiologically acceptable polymers such as polymethylmethacrylate (PMMA), TEFLON (Registered Trade Mark), polycarbonate, polyolefins such as polyethylene, polypropylene, polybutylene, and their mixtures and interpolymers, or a silicone polymer or interpolymer such as are known in the art to be appropriately used in hard contact lenses. PMMA has a long history in ophthalmological usage and consequently is quite desirable for use in these ACICRs. However, another desirable variation is shown in Figures 9A and 9B. In this variation, the added dimension comprises a polymer which is swellable or expands upon continued contact with water. For instance, Figure 9A shows a cross-section of an ACICR having a central portion (76) of a polymer such as PMMA and two regions (78) bonded to the inner and outer periphery of central portion (76). Outer portions (78) may be made of a crosslinked polymeric gel such as polyhydroxyethylmethylacrylate (Poly-HEMA) or polyvinylpyrrolidone (PVP). The extent of crosslinking in these polymers determines the extent to which the polymers will swell upon being exposed to water. In general, the higher the extent of crosslinking, the lower the volume increase upon contact with water. Some materials used in soft contact lenses will contain up to 99% by volume water. In any event, Figure 9A shows the bonded outer portions (78) in their dehydrated condition (if the polymer is not highly crosslinked) and Figure 9B shows those same outlying portions after insertion into the cornea and after they have been allowed to hydrate and swell. This variation of the inventive ACICRs allows the device to be inserted at a much smaller size but allows the ring to swell to correct much larger aberrations.

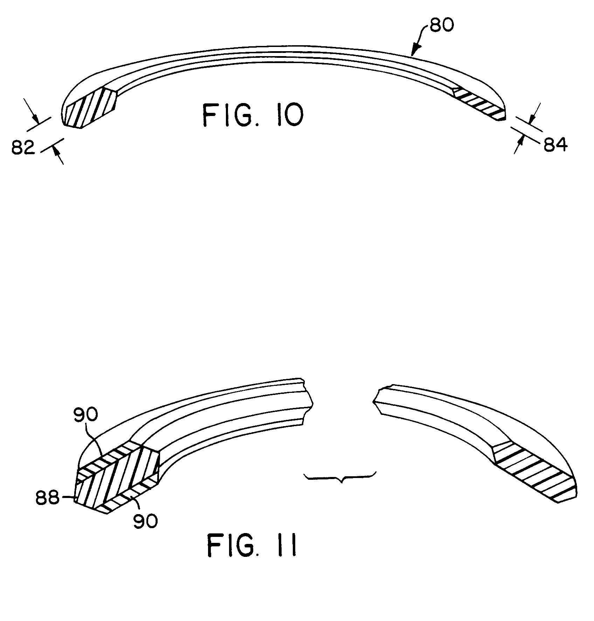

The ACICR (80) shown in Figure 10 is one in which the thickness of at least one portion of the regions is thicker (82) than another thinner portion (84) of the ring. As with the other ACICRs depicted in Figures 3-7, the number of thicker portions of the ring may be one or more depending upon the spherical aberration to be corrected. The typical ACICR likely will have two thicker regions about 180° apart on the ring. The ring may be between about 45° and more than about 140°. The portions of the arc which are thicker are also to be determined depending upon the astigmatism of the eye to be repaired.

Figure 11 shows another variation of the ACICRs in which the ring is a composite assembly. The central portion (88) is of the one or more of the stiff polymers discussed above. The peripheral portions (90) comprise a swellable polymer which swells upon contact with moisture. As with the composite ACICRs above, they may be introduced into the eye prior to hydration, adjusted into proper position, and allowed to hydrate and swell into final shape.

The terms and expressions which have been used in the description above are used as terms of description and not of limitation. There is no intention of excluding equivalents of the features shown or described. It is recognized that one having ordinary skill in this art would perceive equivalents to the inventions claimed below which equivalents would be within the scope of the appended claims.

## Claims

1. A split polymeric ring (50,80) having a cross-section, which ring is suitable for introduction into the stroma of a patient's eye and is adapted to substantially encircle the cornea, said ring having a first end, a second end, and an arcuate body extending therebetween, characterised in that the cross-section of said arcuate body has at least one area (52,60) of comparatively larger dimension.

2. The ring of claim 1, in which the cross section of said arcuate body has two areas (52,60) of comparatively larger dimension.

3. The ring of claim 2, in which the areas (54,60) of larger cross section have transition regions (62) to the areas (64) of smaller dimension.

4. The ring of claim 2, in which the regions of comparatively larger dimension are spaced apart at an angle between 45° and more than about 180°.

5. The ring of claim 2 or claim 3, in which the two areas (60) of comparatively larger dimension are spaced about 180° apart on the ring.

6. The ring of any one of the preceding claims, additionally comprising adjusting means (72) for positioning the ring in the eye.

7. The ring of claim 1 in which the cross section of said arcuate body has more than two areas of comparatively larger cross-section.

8. The ring of claim 2 or claim 3, in which the two areas of comparatively larger dimension are not symmetrical about the ring.

9. The ring of any one of the preceding claims, wherein the ends of the split ring are joinable.

10. The ring of any one of the preceding claims, comprising one or more physiologically acceptable polymers.

11. The ring of claim 10 where the polymer comprises polymethylmethacrylate.

12. The ring of claim 10 additionally comprising a swellable polymer.

13. The ring of claim 12 in which the swellable polymer is selected from polyhydroxyethylmethylacrylate and polyvinylpyrrolidone.

## Patentansprüche

1. Ein geschlitzter, polymerer Ring (50, 80) mit einem Querschnitt, wobei dieser Ring zum Einbringen in das Stroma eines Auges eines Patienten geeignet und dazu befähigt ist, die Cornea im wesentlichen zu umgeben, und wobei dieser Ring ein erstes Ende, ein zweites Ende und einen sich dazwischen erstreckenden, gekrümmten Körper aufweist, **dadurch gekennzeichnet**, daß der Querschnitt des genannten gekrümmten Körpers wenigstens einen Bereich (52, 60) mit einer vergleichsweise größeren Abmessung aufweist.

2. Der Ring nach Anspruch 1, bei welchem der Querschnitt des genannten gekrümmten Körpers zwei Bereiche (52, 60) mit einer vergleichsweise größeren Abmessung aufweist.

3. Der Ring nach Anspruch 2, bei welchem die Bereiche (54, 60) mit größerem Querschnitt Übergangsregionen (62) zu den Bereichen (64) mit kleinerer Abmessung aufweisen.

4. Der Ring nach Anspruch 2, bei welchem die Regionen mit vergleichsweise größerer Abmessung mit einem Winkel zwischen 45 ° und mehr als etwa 180° voneinander beabstandet sind.

5. Der Ring nach Anspruch 2 oder nach Anspruch 3, bei welchem die zwei Bereiche (60) mit vergleichsweise größerer Abmessung mit etwa 180° an dem Ring voneinander beabstandet sind.

6. Der Ring nach einem der vorhergehenden Ansprüche, welcher zusätzlich eine Einstelleinrichtung (72) zum Positionieren des Rings in dem Auge aufweist.

7. Der Ring nach Anspruch 1, bei welchem der Querschnitt des genannten gekrümmten Körpers mehr als zwei Bereiche mit vergleichsweise größerem Querschnitt aufweist.

8. Der Ring nach Anspruch 2 oder nach Anspruch 3, bei welchem die zwei Bereiche mit vergleichsweise größerer Abmessung nicht symmetrisch um den Ring sind.

9. Der Ring nach einem der vorhergehenden Ansprüche, bei welchem die Enden des geschlitzten Rings verbindbar sind.

10. Der Ring nach einem der vorhergehenden Ansprüche, welcher ein oder mehrere physiologisch annehmbare Polymere aufweist.

11. Der Ring nach Anspruch 10, bei welchem das Polymer Polymethylmethacrylat aufweist.

12. Der Ring nach Anspruch 10, welcher zusätzlich ein quellbares Polymer aufweist.

13. Der Ring nach Anspruch 12, bei welchem das quellbare Polymer aus Polyhydroxyethylmethylacrylat und Polyvinylpyrrolidon ausgewählt ist.

## Revendications

1. Anneau polymère tendu (50, 80) ayant une section transversale, cet anneau étant approprié pour l'introduction dans le stroma de l'oeil d'un patient, et étant adapté pour encercler sensiblement la cornée, ledit anneau comportant une première extrémité, une deuxième extrémité, et un corps en forme d'arc s'étendant entre elles, caractérisé en ce que la section transversale dudit corps en forme d'arc comporte au moins une zone (52, 60) de dimension comparativement plus grande.

2. Anneau selon la revendication 1, dans lequel la section transversale dudit corps en forme d'arc comporte deux zones (52, 60) de dimension comparativement plus grande.

3. Anneau selon la revendication 2, dans lequel les zones (54, 60) de section transversale plus grande comportent des régions de transition (62) vers les zones (64) de dimension plus petite.

4. Anneau selon la revendication 2, dans lequel les régions de dimension comparativement plus grande sont espacées l'une de l'autre d'un angle compris entre 45° et plus de 180° environ.

5. Anneau selon la revendication 2 ou la revendication 3, dans lequel les deux zones (60) de dimension comparativement plus grande sont espacées d'environ 180° l'une de l'autre sur l'anneau.

6. Anneau selon l'une quelconque des revendications précédentes, comprenant de plus des moyens de réglage (72) pour positionner l'anneau dans l'oeil.

7. Anneau selon la revendication 1, dans lequel la section transversale dudit corps en forme d'arc comporte plus de deux zones de section transversale comparativement plus grande.

8. Anneau selon la revendication 2 ou la revendication 3, dans lequel les deux zones de dimension comparativement plus grande ne sont pas symétriques autour de l'anneau.

9. Anneau selon l'une quelconque des revendications précédentes, dans lequel les extrémités de l'anneau tendu peuvent être réunies.

10. Anneau selon l'une quelconque des revendications précédentes, comprenant un ou plusieurs polymères physiologiquement acceptables.

11. Anneau selon la revendication 10, dans lequel le polymère comprend du poly(méthacrylate de méthyle).

12. Anneau selon la revendication 10, comprenant de plus un polymère gonflable.

13. Anneau selon la revendication 12, dans lequel le polymère gonflable est choisi parmi le poly(acrylate d'hydroxyéthylméthyle) et la polyvinylpyrrolidone.
